# EUROPEAN PATENT SPECIFICATION

(11) **EP 2 974 721 B1**
(45) Date of publication and mention of the grant of the patent: **12.02.2020**
(21) Application number: 15177007.0
(22) Date of filing: 16.07.2015
(51) Int. Cl.: A61K 9/46, A61K 9/48, A61K 38/28

(54) **A PHARMACEUTICAL COMPOSITION COMPRISING A GAS GENERATING INGREDIENT**
PHARMAZEUTISCHE ZUSAMMENSETZUNG ENTHALTEND EIN GASERZEUGENDEN MITTEL
COMPOSITION PHARMACEUTIQUE COMPRENANT UN AGENT GÉNÉRATEUR DE GAZ

(30) Priority: 18.07.2014 TW 103124722; 02.04.2015 US 201514677832
(43) Date of publication of application: 20.01.2016
(73) Proprietor: Nanomega Medical Corporation, Lake Forest CA 92630 (US)
(72) Inventor: SUNG, Hsing-Wen, 30013 Hsinchu (TW); CHUANG, Er-Yuan, 30013 Hsinchu (TW); LIN, Po-Yen, 30013 Hsinchu (TW); TU, Hosheng, Newport Beach, CA California 92657 (US)
(74) Representative: Lee, Nicholas John

(56) References cited:
- EP-A1- 1 062 952
- WO-A1-90/01329
- WO-A1-2013/059768
- WO-A2-2009/125432
- US-A- 3 961 041
- US-A- 4 849 227
- US-B1- 8 574 544
- SADEGHI A M M ET AL: "Development of a Gas Empowered Drug Delivery system for peptide delivery in the small intestine", JOURNAL OF CONTROLLED RELEASE, ELSEVIER, AMSTERDAM, NL, vol. 134, no. 1, 20 February 2009 (2009-02-20), pages 11-17, XP025908820, ISSN: 0168-3659, DOI: 10.1016/J.JCONREL.2008.10.012 [retrieved on 2008-10-26]
- ER-YUAN CHUANG ET AL: "Self-assembling bubble carriers for oral protein delivery", BIOMATERIALS, vol. 64, 1 September 2015 (2015-09-01), pages 115-124, XP055229122, GB ISSN: 0142-9612, DOI: 10.1016/j.biomaterials.2015.06.035

## Description

### FIELD OF THE INVENTION

The present invention is related to a pharmaceutical composition, and to this composition for use in the treatment of diseases or disorders of an animal subject, wherein the composition comprises at least one bioactive agent and nano/micro bubble forming agent, with enhanced efficacy for oral drug delivery.

### BACKGROUND OF THE INVENTION

Unlike small molecule drugs that are manufactured by synthesis, biologics are synthesized in vivo by bacterial or mammalian cells as a platform. However, hydrophilic macromolecule drugs such as proteins, peptides, polysaccharides and nucleic acids, due to their structural properties and instability in the gastric acid, often must be prepared as injection dosage. Here disclosed is an oral drug delivery system with a pharmaceutical composition of gas generating microspheres/nanospheres or effervescent agents for enhanced drug release in the small intestine tract of an animal subject.

EP 1 062 952 A1 discloses enteric coated tablets and capsules comprising glycyrrhizin, sodium caprate, sodium hydrogen carbonate and citric anhydride.

### SUMMARY OF THE INVENTION

In the first place, the present invention concerns a pharmaceutical composition suitable for oral administration to an animal subject, comprising a gas generating ingredient, a surfactant, an acid initiator and at least one bioactive agent, wherein said pharmaceutical composition is loaded within an enteric-coated capsule, said gas generating ingredient is selected from the group consisting of ammonium bicarbonate, sodium bicarbonate, potassium bicarbonate, and calcium bicarbonate, and said acid initiator reacts with said gas generating ingredient in an intestinal tract of the animal subject to produce a gas which is carbon dioxide upon contacting water, and said acid initiator is an anhydride of complexone, said complexone being selected from the group consisting of DTPA (diethylene triamine pentaacetic acid), EDTA (ethylene diamine tetra acetate), IDA (iminodiacetic acid), NTA (nitrilotriacetic acid), EGTA (ethylene glycol tetraacetic acid), BAPTA (1,2-bis(o-aminophenoxy)ethane-N,N,N',N'-tetraacetic acid), DOTA (1,4,7,10-tetraazacyclododecane-N,N',N,N' tetraacetic acid), and NOTA (2,2',2"-(1,4,7-triazonane-1,4,7-triyl)triacetic acid). Secondly, the invention concerns the above composition for use in medicine. The scope of the invention is defined by the claims. Any references in the description to methods of treatment refer to the compounds, pharmaceutical compositions and medicaments of the present invention for use in a method for treatment of the human (or animal) body by therapy.

Herein provided is a method for oral administration of an active ingredient by using nano/micro bubbles-generating ingredient as a vehicle, which can be effectively used for drug delivery, release and absorption in animal subjects, including humans.

In particular, herein provided is a method for oral administration of at least one active ingredient to a subject whereby, after oral delivery, a plurality of bubbles containing the active ingredient are formed in the target small intestine of the subject. The method for oral administration of an active ingredient(s) to a subject includes administering orally to the subject an effective amount of the pharmaceutical composition described herein. According to the present invention, the pharmaceutical composition comprises a gas generating ingredient, a surfactant, an acid initiator and at least one bioactive agent. The gas generating agent also commonly refers to a bubbling agent, bubbles-generating agent, gas producing agent, effervescent agent, or foaming agent; all above-mentioned terms herein are interchangeable in this application. The active ingredient comprises, but not limited to, a nucleic acid, a peptide, protein, and the like. The active ingredient includes either a hydrophilic or hydrophobic active agent or a combination of hydrophilic and hydrophobic agents. The pharmaceutical composition is loaded within an enteric-coated capsule, which encapsulates one or more of the ingredients of the pharmaceutical composition in their solid forms. Embodiments wherein the enteric coating layer is an enteric-coated tablet or pill, which covers the entire surfaces of the ingredients mass of the pharmaceutical composition in their solid tablet or pill forms, are not according to the invention. When the acidic component of the pharmaceutical composition is dissolved upon contacting water or fluid in the small intestine to react with the effervescent ingredient for generating carbon dioxide, a plurality of carbon dioxide bubbles (or gas cores) containing the active ingredient at the bubble's surface is formed. In some embodiment, the active ingredient is embedded in a gap formed between an inner layer and an outer layer of a double-layer structure formed by the surfactant ingredient of the pharmaceutical composition on the carbon dioxide gas core. In another embodiment, the active ingredient is adsorbed on or associated with the bubbles or gas cores.

Some aspects of the invention provide a pharmaceutical composition as claimed for oral delivery to an animal subject, comprising a gas generating ingredient and at least one bioactive agent, wherein the pharmaceutical composition is loaded within an enteric-coated capsule. The gas generating ingredient is selected from the group consisting of ammonium bicarbonate, sodium bicarbonate, potassium bicarbonate, and calcium bicarbonate. The pharmaceutical composition further comprises an acid initiator. The acid initiator is an anhydride of complexone. The complexone is selected from the group consisting of DTPA (diethylene triamine pentaacetic acid), EDTA (ethylene diamine tetra acetate), IDA (iminodiacetic acid), NTA (nitrilotriacetic acid), EGTA (ethylene glycol tetraacetic acid), BAPTA (1,2-bis(o-aminophenoxy)ethane-N,N,N',N'-tetraacetic acid), DOTA (1,4,7,10-tetraazacyclododecane-N,N',N,N'-tetraacetic acid), and NOTA (2,2',2"-(1,4,7-triazonane-1,4,7-triyl)triacetic acid). The acid initiator reacts with the gas generating ingredient in an intestinal tract of the animal subject to produce a gas upon contacting water, wherein the gas is carbon dioxide.

Some aspects of the invention provide a pharmaceutical composition as claimed for oral delivery to an animal subject, comprising a gas generating ingredient and at least one bioactive agent, wherein the pharmaceutical composition is loaded within an enteric-coated capsule, wherein the capsule is made of gelatin or methyl cellulose selected from the group consisting of hydroxypropyl methyl cellulose (HPMC), carboxymethyl cellulose, hydroxyethyl methyl cellulose (HEMC), and methyl cellulose derivatives. The pharmaceutical composition of the present invention may further comprise at least one excipient. The at least one excipient is preferably selected from the group consisting of a pharmaceutically acceptable carrier, diluent, desiccant, solubilizer, absorption enhancer, and emulsifier. The absorption enhancer is preferably selected from the group consisting of bile salts, medium-chain fatty acids, phosphate esters, chitosan, and chitosan derivatives. Preferably, the surfactant is sodium dodecyl sulfate, polyoxyethylene sorbitan monostearate, or sodium dodecyl benzene sulfonate. Preferably, the surfactant is an anionic surfactant, cationic surfactant, amphoteric or non-ionic surfactant.

Some aspects of the invention provide a pharmaceutical composition as claimed for oral delivery to an animal subject, comprising a gas generating ingredient and at least one bioactive agent, wherein the pharmaceutical composition is loaded within an enteric-coated capsule, wherein the at least one bioactive agent is an anti-diabetic compound, wherein the anti-diabetic compound is selected from the group consisting of insulin, an insulin analog, GLP-1, a GLP-1 analog, an insulin sensitizer, an insulin secretagogue, an inhibitor of dipeptidyl peptidase 4, metformin, alpha-glucosidase inhibitors, amylin analog, sodium-glucose co-transporter type 2 (SGLT2) inhibitors, benfluorex, tolrestat, mitofusin 1, mitofusin 2, and combinations thereof. The at least one bioactive agent may be selected from the group consisting of proteins, peptides, nucleosides, nucleotides, antiviral agents, antineoplastic agents, antibiotics, oxygen-enriching agent, oxygen-containing agent, growth hormone, human growth hormone, heparin, low molecular weight heparin, anti-coagulating agents, anti-hemophilic factor, anti-epileptic drug, Alzheimer's antagonist, anti-inflammatory drugs, calcitonin, cyclosporin, erythropoietin, erythropoiesis-stimulating agent, oxytocin, tyrosine, enkephalin, tyrotropin releasing hormone, follicle stimulating hormone, luteinizing hormone, vasopressin and vasopressin analogs, catalase, superoxide dismutase, interleukin-11, interferon, colony stimulating factor, granulocyte colony-stimulating factor, tumor necrosis factor, tumor necrosis factor inhibitor, melanocyte-stimulating hormone, antibodies, and monoclonal antibodies.

Some aspects of the invention provide a drug delivery bubble, comprising: a gas core; surfactants surrounding the gas core and forming a double-layer structure, wherein the double-layer structure is provided with an inner layer and an outer layer formed by the surfactants upon the gas core. Preferably, an active ingredient (also known as a bioactive agent or API) is embedded in a gap formed between the inner layer and the outer layer of the double-layer bubble structure. Preferably, the active ingredient is a hydrophilic or hydrophobic substance. The outer layer may cover a portion of the bubble gas core, whereby the bubble gas core surface herein is the inner layer. The outer layer and/or inner layer may cover at least a portion of the bubble gas core surface. An active ingredient can be adsorbed on the surface of the gas core, on the inner layer, or on the outer layer. Preferably, each of the surfactants has a hydrophilic head and a hydrophobic tail, the hydrophobic tails of the surfactants of the inner layer face the gas core and the hydrophobic tails of the surfactants of the outer layer face towards the outer side of the bubbles, and the hydrophilic heads of surfactants of the inner layer and the hydrophilic heads of surfactants of the outer layer are arranged opposite to each other to form the double-layer structure. The surfactants may comprise anionic surfactants, cationic surfactants, amphoteric or non-ionic surfactants. Further, the surfactants comprise sodium dodecyl sulfate, polyoxyethylene sorbitan monostearate, sodium laureth sulfate or sodium dodecyl benzene sulfonate.

Preferably the diameter of the bubbles of the invention may range between 4.5 nm and 100 µm, preferably ranges between 10 nm and 10 µm. Preferably, the diameter of the bubbles of the present invention is 4.5 nm or smaller. It is deemed novel and unobvious for a pharmaceutical composition comprising an effervescent ingredient and at least one active ingredient in an enteric-coated capsule from prior arts.

Some aspects of the invention provide a pharmaceutical composition as claimed for use in a method for oral administration of an active ingredient to an animal subject, whereby a plurality of bubbles containing the active ingredient are formed in a small intestine of the subject, wherein the method comprises administering orally to the subject an effective amount of a pharmaceutical composition comprising: a drug layer, comprising: the active ingredient, comprising a nucleic acid, a peptide and a protein; a surfactant; an acidic component; and an effervescent ingredient; and an enteric coating layer encapsulating the drug layer, whereby the acidic component of the drug layer is dissolved in the small intestine to react with the effervescent ingredient for generating carbon dioxide and the plurality of bubbles containing the active ingredient, and the active ingredient is embedded in a gap formed between an inner layer and an outer layer of a double-layer structure formed by the surfactant. The pharmaceutical composition is a capsule.

### BRIEF DESCRIPTION OF THE DRAWINGS

Additional objects and features of the present invention will become more apparent and the disclosure itself will be best understood from the following Detailed Description of the Exemplary Embodiments, when read with reference to the accompanying drawings.
Fig. 1 is a schematic diagram illustrating the nano/micro bubbles according to one embodiment of the present invention;
Fig. 2 to Fig. 4 are experimental data of an embodiment of the present invention;
Fig. 5 show (a) plasma insulin level *vs.* time profiles and (b) blood glucose change *vs.* time profiles of diabetic rats treated with various insulin formulations. Oral: oral administration of test capsules and SC: subcutaneous injection.
Fig. 6a is a schematic diagram illustrating the nano/micro bubbles according to one embodiment of the present invention.
Fig. 6b is a partial enlarged view of Fig. 6a.
Fig. 7 show schematic illustrations showing the formation and expansion of bubble carriers (see subfigures a, b, and c) developed from the orally administered gelatin capsule and their mechanism for delivering insulin across the epithelial barrier. TJ: tight junction; AJ: adherens junction.
Fig. 8 show (a) results of dissolution and disintegration of the test capsules with or without SBC evaluated *in vitro* under different pH levels to simulate the pH environments in the GI tract; (b) ultrasound images showing the generation of CO₂ bubbles.
Fig. 9 show fluorescence images revealing encapsulation of both (a) FITC-insulin and (b) Cy3-DTPA within the water film of the bubble carriers; (c) corresponding schematic illustrations displaying the bubble carriers produced upon exposure to water; (d) SAXS profiles showing structural changes in the formation and expansion of bubbles.
Fig. 10 show results of insulin degradation in the presence or absence of DTPA or SDS in proximal intestinal segments freshly isolated from rats. N.S.: not significant; *: statistically significant (*P* < 0.05).
Fig. 11 show sequential plain X-ray and X-ray/scintigraphy images of test rats orally treated with the capsules containing barium sulfate and ¹²³I-insulin (white arrow) with or without foaming agent.
Fig. 12 show chemical structures of the major ingredients for the nanobubbles forming system of the present invention.

### DETAILED DESCRIPTION OF THE EXEMPLARY EMBODIMENTS

The present invention described below relates particularly to the pharmaceutical composition as claimed. It also refers to this composition for use in medicine. While the description sets forth various specific details, it should be understood that the description is illustrative only and should not be construed in any way as limiting the invention. Furthermore, various applications of the invention, and modifications thereto, which may occur to those who are skilled in the art, are also encompassed by the general concepts described below.

Successful oral delivery of therapeutic proteins such as insulin can greatly improve the quality of life for patients. This system of the present invention comprises a bubble-generating system by loading one or more of the ingredients such as: diethylene triamine pentaacetic acid (DTPA) dianhydride, a foaming agent (sodium bicarbonate; SBC), a surfactant (sodium dodecyl sulfate; SDS), and a protein/peptide drug (for example, insulin) in an enteric-coated capsule. Following oral administration to diabetic subjects, the intestinal fluid, at a pH above the pH of the enteric-coating polymer on capsules, passes through the enteric layer. The fluid then penetrates and/or saturates the powder mixture inside the capsules; concomitantly, DTPA dianhydride (or an acid initiator) produces an acidic environment, while SBC decomposes to form CO₂ bubbles or gas at an acidic pH environment. The pressure of the generated gas ruptures the capsule further, enhancing a quick release of its loaded contents toward the intestinal wall.

The gas bubbles grow, preferably from nanoscale to microscale among the surfactant molecules (SDS), owing to the expansion and volume increase of the generated CO₂. The walls or surfaces of the CO₂ bubbles consist of a self-assembled film of water (or fluid) that is in nanoscale and may serve as a colloidal carrier to transport a bioactive agent (for example, insulin or GLP-1) and DTPA. The insulin bioactive agent and/or DTPA may be sandwiched between two layers of SDS or other appropriate surfactant. The grown gas bubbles continue to expand during their transit in the intestinal tract until they bump into the wall and burst, releasing their transported bioactive agent, DTPA, and/or SDS into the mucosal layer. The released DTPA and SDS, individually or in combination, function as protease inhibitors to protect the bioactive insulin molecules as well as function as absorption enhancers to augment their epithelial permeability and eventual absorption into systemic circulation.

It is one object of the present invention to provide a novel, unobvious, unique system and methods of preparation for protein/peptide drug or bioactive agent delivery using a quick and effective delivery of a drug pharmaceutical composition via enteric-coated capsules in an oral drug delivery system. The novel drug carrier system involves a foaming agent (for example sodium bicarbonate; SBC) that generates CO₂ bubbles upon exposure to an acidic aqueous environment. The walls of these CO₂ bubbles (bubble also known as a gas core in this application) preferably consist of a nanoscale self-assembled film of water that can be sandwiched between two layers of surfactant molecules to form a colloidal carrier for transporting protein drugs or other bioactive agents. Absorption can be enhanced by formulating the powder drug as a solubilizable component within a colloidal dispersion. This bubble generating system can be prepared by mixing diethylene triamine pentaacetic acid (DTPA) dianhydride, SBC, a surfactant (sodium dodecyl sulfate; SDS), and a protein drug (insulin). The DTPA dianhydride is an acidic oxide that dissolves in water to form an acid (for example, DTPA) solution. When reacting with the acid, SBC produces a bubbly CO₂ gas.

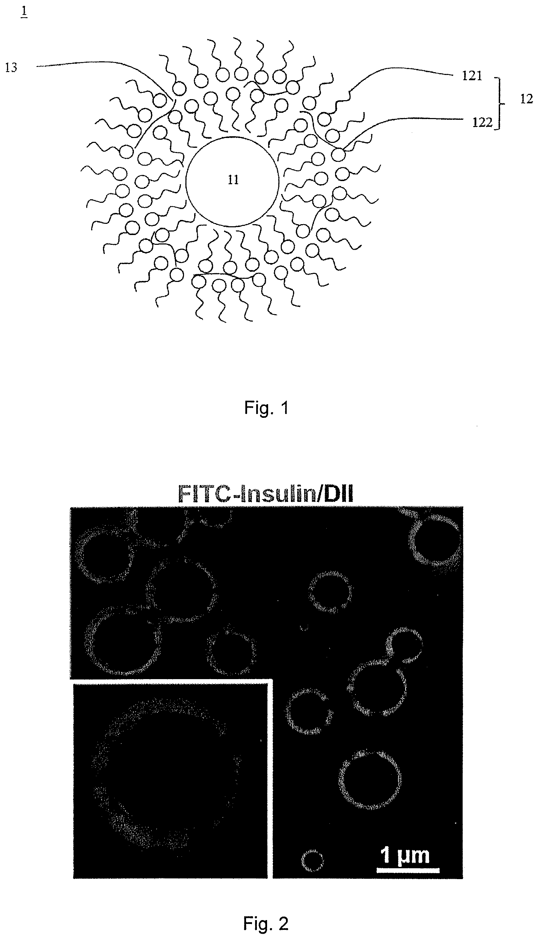

Refer to Fig. 1, which is a schematic diagram illustrating the nano/micro bubbles according to one embodiment of the present invention, wherein surfactants 12 surround the gas core 11 and form a double-layer structure. Each surfactant 12 has a hydrophilic head 122 and a hydrophobic tail 121 and the double-layer structure is comprised with an inner layer and an outer layer formed by the surfactant 12 on top of the gas core 11. The surfactant 12 in the inner layer faces the gas core 11 with its hydrophobic tail 121 and the surfactant 12 in the outer layer towards the outer side of the nano/micro bubble 1 with its hydrophobic tail 121. The surfactant 12 in the inner layer and the surfactant 12 in the outer layer of the double-layer structure align to each other with their hydrophilic heads to form the double-layer structure. The active ingredient 13 is embedded in a gap formed between the inner layer and the outer layer of the bilayer structure. The gas core 11 of carbon dioxide or other gases may be encapsulated by the surfactant 12.

The particle diameter of the nano/micro bubbles of the present invention mainly depends on the size of the gas core and thus may vary in large ranges. The particle diameter of nano/micro bubbles may mainly range between 4.5 nm and 100 µm, preferably range between 10 nm and 10 µm. The diameter of the bubbles of the present invention may be 4.5 nm or smaller.

It should be noted that the existence of the gas core 11 in the nano/micro bubbles of the present invention is quite important. The gas core is novel and unobvious when compared to the center of conventional liposomes or cell membranes that is filled with liquid. Therefore, the nano/micro bubbles of the present invention may be distinguished from conventional liposomes or cell membranes. Some aspects of the invention provide a pharmaceutical composition as claimed comprising: at least one active pharmaceutical ingredient, at least one effervescent ingredient, at least one surfactant ingredient, and at least one acidic component (or acid initiator) ingredient, wherein all ingredients are encapsulated in an enteric-coated capsule. Upon oral capsule delivery to a patient's intestinal tract and the capsule contacting intestinal fluid, a plurality of bubbles (nano/micro bubbles) are formed, wherein the inner space of bubbles is substantially filled with carbon dioxide in its gaseous state. Effervescence is the escape of gas from an aqueous solution and the foaming or fizzing that results from a release of the gas. An effervescent ingredient or agent is a substance that can cause the phenomenon of effervescence by producing and releasing the gas.

Furthermore, it should be noted that the double layer structure of the nano/micro bubbles formed by the surfactant 12 may be in the opposite way from the lipid bilayer provided in conventional liposomes or cell membrane. To be specific, the hydrophobic tails 121 of the surfactant 12 may face the gas core 11 and towards the outer side of the nano/micro bubbles, and the hydrophilic heads 122 may be arranged opposite to each other to form the double-layer structure so as to maintain hydrophobicity against water. However, the hydrophilic heads of the conventional lipid bilayers structure face the center and towards the outer side, and arranged opposite to each other with hydrophobic tails to form a bilayer structure.

In general, surfactant 12 can be classified as anionic surfactants, cationic surfactants, amphoteric or non-ionic surfactants. Anionic surfactants may include, but are not limited to, alkyl sulfates, sodium dodecyl sulfate, n-dodecyl benzene sulfonate, sodium laureth sulfate and so on. The cationic surfactants may include, but are not limited to, polyoxyethylene lauryl dimethylamine salts and so on. The nonionic surfactant may include, but are not limited to, polyoxyethylene sorbitan monostearate and so on.

As shown in Fig. 1, active ingredient 13 may be embedded in the gap formed between hydrophilic heads of surfactant arranged opposite to each other; therefore, the size of the active ingredient 13 is not particularly limited. The active ingredient 13 may be small molecular drug or biological macromolecules. The active ingredient 13 may comprise nucleic acid, peptide or protein. Here, the nucleic acid comprises deoxyribose nucleic acid (DNA) or ribose nucleic acid (RNA). Protein may comprise an antibody or a protein drug. Protein drugs may include without be limited to, insulin, erythropoietin (EPO) or interferon and so on.

The active ingredient 13 may be hydrophilic so as to increase the hydrophilic interaction between the active ingredient and the hydrophilic head of the surfactant. Hydrophilic active ingredient 13 may be, but are not limited to, insulin and nucleic acids such as DNA, and so on. The surfactant may be chosen based on electric charge of the active ingredient 13 so as to increase the binding affinity between the active ingredient and surfactant. In the case of nucleic acids such as DNA as the active ingredient, for example, since nucleic acids are negatively charged, some cationic surfactants that are positively charged may be chosen so as to enhance the binding affinity between the active ingredient and surfactant.

The surfactant may be chosen based on electric charge of the active ingredient 13 so as to increase the binding affinity between the active ingredient and surfactant. In the case of nucleic acids like DNA as the active ingredient, for example, since nucleic acids are negatively charged, some cationic surfactants that are positively charged may be chosen so as to enhance the binding affinity between the active ingredient and surfactant. Some aspects of the invention provide a pharmaceutical composition as claimed for oral delivery to an animal subject, comprising a gas generating ingredient, a surfactant, an acid initiator, and at least one bioactive agent, wherein the pharmaceutical composition is loaded within an enteric-coated capsule, and wherein the charge of the surfactant is opposite to the charge of the at least one bioactive agent to enhance binding affinity between the at least one bioactive agent and the surfactant.

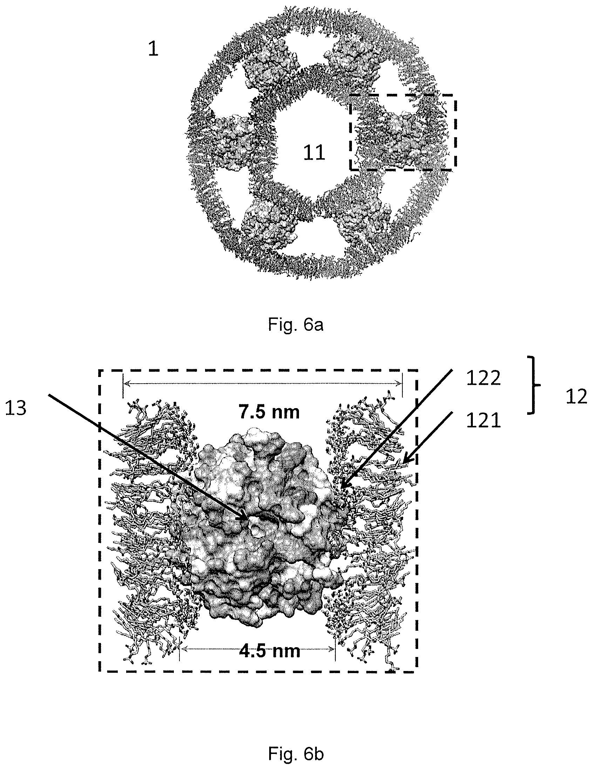

Further referring to Figs. 6a and 6b of the present invention, which are schematic molecular simulation illustrating the nano/micro bubbles of the present invention, wherein Fig. 6b is an enlarged partial view of Fig. 6a. As shown in the example of the diagram, the gap formed between the inner layer and the outer layer of the double layer structure may be about 4.5 nm, and the width of the double-layer structure may be about 7.5 nm. Here, the active ingredient 13 may be insulin that is embedded in the formed gap.

The pharmaceutical composition comprises a surfactant, an effervescent ingredient and an active ingredient. The effervescent ingredient is able to react with an acid to generate carbon dioxide. The effervescent ingredient comprises ammonium bicarbonate, sodium bicarbonate, potassium bicarbonate or calcium bicarbonate. By exposing the formulations of the present invention to the acidic environment, carbon dioxide is generated by the reaction of the effervescent ingredient and the acid irreversibly. As carbon dioxide is generated in a "burst" way, the surfactant of the formulation thus encapsulates the carbon dioxide gas core to form the double-layer structure of the present invention on at least a portion of the carbon dioxide core and the active ingredient is located in the gap or surfaces of the nano/micro bubbles of the present invention.

The pharmaceutical composition of the present invention comprises an acidic ingredient, which may be dissociated or hydrolyzed in the water to form an acid, thereby enabling the effervescent ingredient to generate carbon dioxide in the water. The formulation of the present invention can produce a local acidic environment as described above and functions in a neutral or alkaline environment to generate nano/micro bubbles of the present invention. The acidic ingredient is as claimed.

The ratio of the acidic ingredient, effervescent ingredient and surfactant in the formulation of the present invention may be easily modified for those skilled in the art. For example, the molar ratio of the acidic ingredient, effervescent ingredient and surfactant in one example is 5:21:6 so as to generate carbon dioxide gas from the reaction of the acidic ingredient and effervescent ingredient in the presence of water.

The formulation or pharmaceutical composition of the present invention comprises an enteric coating layer encapsulating the powder mixture ingredients. The enteric coating layer may be made of, but are not limited to, (methyl) acrylic acid copolymer, hydroxypropyl cellulose phthalate ester, hydroxypropyl cellulose acetate ester, hydroxypropyl cellulose acetate succinate ester or carboxymethyl ethyl cellulose. The enteric polymer may be sprayed onto the surface of a capsule to manufacture the enteric-coated capsule containing the active pharmaceutical ingredient, a surfactant ingredient, an acidic component ingredient, an effervescent ingredient, and any other compound.

The drug ingredients may be filled into gelatin capsules, and the outer surface of the capsule may then be coated with EUDRAGIT® by coating techniques so as to avoid damage caused by gastric acid. The ingredients of the pharmaceutical composition are maintained intact until arriving at the small intestine, where the effervescent ingredient generates carbon dioxide by reacting with acidic ingredient in the water (for example, intestinal fluid). The pharmaceutical composition of the present invention may be utilized to produce local acidic environment in an alkaline environment (e.g., the small intestine) so that the above reaction may take place to generate the nano/micro bubbles of the present invention.

In terms of acceptable excipients, the pharmaceutical composition may comprise any optional additives, such as pharmaceutically acceptable carrier or diluent, flavors, sweeteners, preservatives, antioxidants, wetting agents, buffering agents, release controlling component, dyes, adhesives, suspending agents, dispersing agents, coloring agents, disintegrating agents, excipients, film forming agents, lubricants, plasticizing agents, oil or any two or more combinations of the above.

Suitable pharmaceutically acceptable carrier or diluent include, but are not limited to, ethanol, water, glycerol, propylene glycol or glycerol, diethylene glycol monoethyl ether, vitamin A and E oils, mineral oil, PPG2 myristyl propionate salt, potassium phosphate or silica. Suitable lubricants may be oleate, sodium stearate, sodium lauryl fumarate, magnesium stearate, sodium benzoate, sodium acetate and sodium chloride. Suitable suspending agents may be bentonite, ethyl, isostearyl alcohol, polyoxy ethylene sorbitol and sorbitan esters, microcrystalline cellulose, aluminum metahydroxide, agar and tragacanth, or the mixture of two or more of these substances. Suitable dispersing and suspending agents may be synthetic and natural gums, such as vegetable gum, tragacanth, acacia, alginate, dextran, sodium carboxymethyl cellulose, methyl cellulose, polyvinyl-pyrrolidone and gelatin. Suitable film-forming agents include hydroxypropyl methyl cellulose, ethyl cellulose and polymethacrylates. Suitable plasticizing agents include polyethylene glycol having different molecular weights (e.g. 200-8000 Da), polypropylene glycol and triethyl citrate. Suitable coloring agent is ferric oxide, titanium dioxide and natural and synthetic colors. Examples of additional additives include sorbitol, talc or stearic acid. In general, the ingredients of the pharmaceutical composition are in their solid form when loaded in a capsule.

A method for oral administration of an active ingredient to a subject is also described whereby a plurality of bubbles containing the active ingredients are formed in the small intestine of a subject. The method for oral administration of an active ingredient to a subject includes administering orally to the subject an effective amount of the above-mentioned pharmaceutical composition whereby the acidic component of the drug pharmaceutical composition is dissolved in the small intestine to react with the effervescent ingredient for generating carbon dioxide and the plurality of bubbles containing the active ingredient. Preferably, the active ingredient is embedded in a gap formed between an inner layer and an outer layer of an double-layer structure formed by the surfactant.

Preferably, the method is used for treating diabetes and the active ingredient is insulin. As used herein, the terms "patient," "host," "user," and "subject" refer to any human or animal subject and are not intended to limit the systems or methods to human use, although use of the subject invention in a human patient represents a preferred embodiment. As mentioned above, the nano/micro bubbles of the present invention are preferably generated in the intestinal tract. Once nano/micro bubbles are in contact with the epithelial cells in the intestinal epidermis, the surfactant can promote the paracellular pathway as well as the transcellular pathway such that the active ingredient of the present invention can be delivered to target sites of the body circulation.

The present invention is further illustrated by the following working examples, which should not be construed as further limiting.

**Preparation of nano/micro bubble formulation.** One example of surfactants is SDS, one example of the acidic ingredient is DTPA dianhydride (diethylenetriaminepentaacetic dianhydride), one example of the effervescent ingredient is baking soda (sodium bicarbonate, SBC), one example of the active ingredient is insulin, and one example of the enteric coating material is EUDRAGIT® L100-55. One preparation is listed as following:
1. SDS (7 mg), DTPA dianhydride (14 mg), SBC (7 mg), insulin (5 IU / Kg) were uniformly mixed and filled into gelatin capsules.
2. The prepared gelatin capsules as described above are coated with EUDRAGIT® L100-55 (10%) and then dried.

**Verification of nano/micro bubbles.** Referring to Fig. 2, the insulin was labeled with FITC, and the hydrophobic tail of SDS was stained with a fluorescent lipophilic cationic indocarbocyanine dye DII (1,1'-dioctadecyl-3,3,3',3'-tetramethylindocarbocyanine perchlorate), respectively. The obtained fluorescence images proved that insulin does exist in the nano/micro bubbles of the present invention and is located at the gap of the double-layer structure formed by the surfactant via fluorescence imaging techniques. This indicates that the active ingredient is well embedded in the nano/micro bubbles of the present invention.

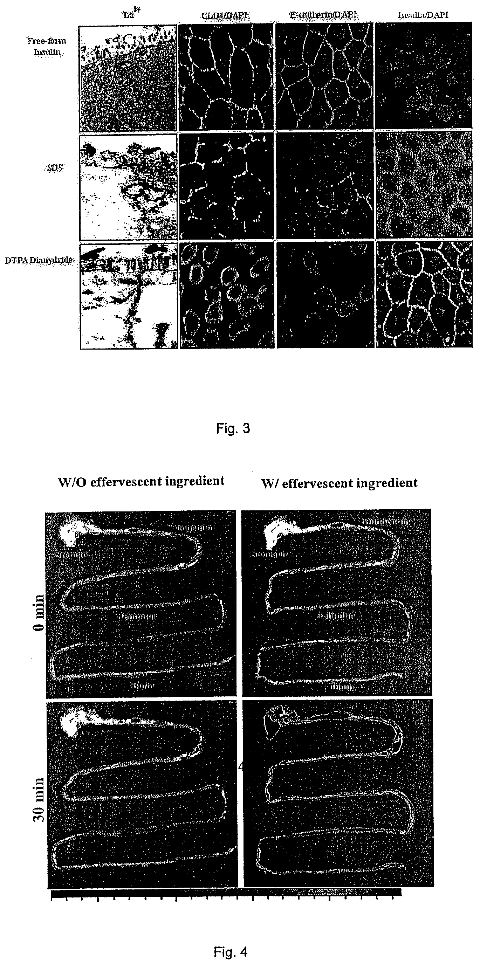

**Cell images of cells bound with nano/micro bubbles.** Referring to Fig. 3, which shows the images under the microscope after cells were co-cultured with the various components, wherein the cells were stained with Cld 4 (tight junction), E-cadherin (adherin junction) and DAPI (nuclear). The results showed that DTPA dianhydride can promote transmission of FITC-insulin via paracellular pathway; SDS can promote transmission of FITC-insulin via both of paracellular pathway and transcellular pathway.

**In vivo cell images with nano/micro bubbles.** Referring to Fig. 4, which shows the effects of nano/micro bubbles within the intestinal tract. The distribution of the insulin can only be imaged and identified at local areas in the group without effervescent ingredient; the group with the effervescent ingredients of the present invention has demonstrated release of insulin (as imaged) at the middle and end section of the small intestine system. The nano/micro bubbles burst leads to the release of insulin with aid of the effervescent ingredients, resulting in better release performance (and subsequently enhanced intestinal absorption and permeation) in comparison to the control group having no effervescent ingredients.

**Anti-diabetic Drugs.** The anti-diabetic drugs (a bioactive agent in the present invention) are broadly categorized herein as insulin/insulin analogs and non-insulin anti-diabetic drugs. The non-insulin anti-diabetic drugs may include, but not limited to, insulin sensitizers, such as biguanides (for example, metformin, buformin, phenoformin, and the like), thiazolidinedione (TZDs; for example, pioglitazone, rivoglitazone, rosiglitazone, troglitazone, and the like), and dual PPAR agonists (for example aleglitazar, muraglitazar, tesaglitazar, and the like; PPAR is abbreviation for peroxisome proliferator-activated receptor). The non-insulin anti-diabetic drugs may also include, but not limited to, secretagogues, such as sulfonylureas (for example, carbutamide, chlopropamide, gliclazide, tolbutamide, tolazamide, glipizide, glibenclamide, gliquidone, glyclopyramide, glimepiride, and the like), meglitinides (for example, nateglinide, repaglinide, mitiglinide, and the like), GLP-1 analogs (for example, exenatide, liraglutide, albiglutide, taspoglutide, and the like), and DPP-4 inhibitors (for example, alogliptin, linagliptin, saxagliptin, sitagliptin, vildagliptin, and the like; DPP-4 is abbreviation for inhibitor of dipeptidyl peptidase 4).

Further, the non-insulin anti-diabetic drugs may include, but not limited to, alpha-glucosidase inhibitors (for example, acarbose, miglitol, voglibose, and the like), amylin analog (for example, pramlintide and the like), SGLT2 inhibitor (for example, dapagliflozin, remogliflozin, sergliflozin, and the like), benfluorex, and tolrestat. Here, the sodium-glucose co-transporter type 2 (SGLT2) is a 672 amino acid high-capacity low-affinity transporter expressed in the S1 segment of the proximal tubule which is believed to mediate the majority of renal glucose reabsorption. Some aspects of the invention provide a combination capsule comprising two or more of bioactive pharmaceutical ingredients and at least one effervescent ingredient.

The capsule contains a bubbling agent and may contain a solubilizer, and/or emulsifier, and/or other pharmacopoeial excipients, such as Generally Recognized as Safe (GRAS). GRAS is a United States of America Food and Drug Administration (FDA) designation that a chemical or substance added to food is considered safe by experts, and therefore exempted from the usual Federal Food, Drug, and Cosmetic Act (FFDCA) food additive tolerance requirements. The bubbling agent is the agent that emits carbon dioxide gas when contacting liquid with a purpose to burst the capsule or promote intimate contact of the capsule payload with the surrounding material outside of the capsule. For example, reaction of sodium bicarbonate and an acid to give a salt and carbonic acid, which readily decomposes to carbon dioxide and water. A bubbling agent may include a sodium bicarbonate/citric acid mixture, Ac-Di-Sol, and the like. The chemical Ac-Di-Sol has the IUPAC name of acetic acid, 2,3,4,5,6-pentahydroxyhexanal, sodium and a chemical formula of C₈H₁₆NaO₈. The bubbling agents of the invention are selected from the group consisting of ammonium bicarbonate, sodium bicarbonate, potassium bicarbonate, and calcium bicarbonate.

An emulsifier is a substance that stabilizes an emulsion by increasing its kinetic stability. One class of emulsifiers is known as surface active substances, or surfactants. Detergents are another class of surfactant emulsifier, and will physically interact with both oil and water, thus stabilizing the interface between oil or water droplets in suspension. The most popular emulsions are non-ionic because they have low toxicity. Cationic emulsions may also be used herein because of their antimicrobial properties.

The preferred embodiments of the present invention described below relate particularly to the preparation of a pharmaceutical composition and a system of loading the composition within an enteric-coated capsule. While the description sets forth various embodiment specific details, it should be understood that the description is illustrative only and should not be construed in any way as limiting the invention.

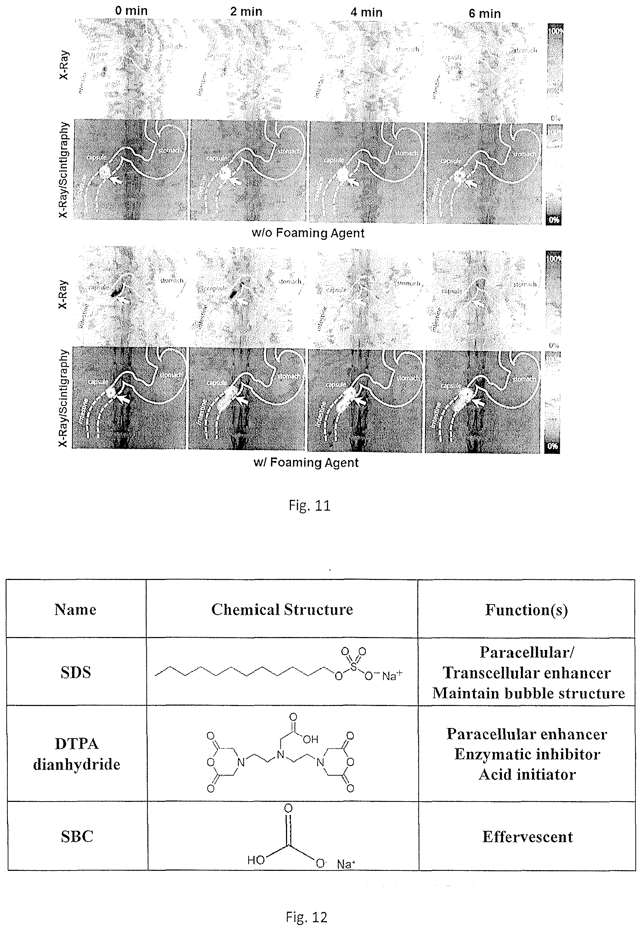

**Preparation of test capsules having the pharmaceutical composition.** In one example, hard gelatin capsules were manually filled with a mixture of SBC (8.0 mg), DTPA dianhydride (8.0 mg), SDS (4.0 mg), and insulin (30.0 IU/Kg) according to manufacturer instructions. The other ratios of ingredients mixture were also studied with similar enhanced bubble-assisted insulin release when compared to the control capsules without effervescent ingredients. The capsules without SBC were used as a control. The prepared capsules were immersed in a methanol solution of Eudragit® L100-55 (15% w/v) and then dried at room temperature using an air-blower. This procedure was preferably repeated three times. As an illustration, Fig. 12 shows the chemical structures of the ingredients for the nanobubbles forming system of the present invention.

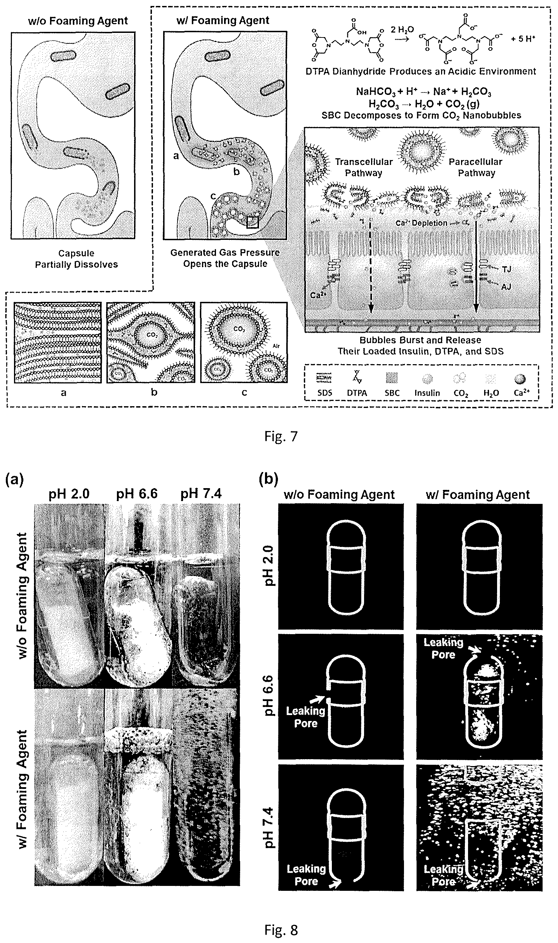

Fig. 7 shows a scheme of this bubble carrier system and how it works. When the water that has passed through the gelatin capsule contacts and saturates the powder mixture, nanosized CO₂ bubbles are generated. As the gas pressure ruptures the capsule, the payload is quickly and overwhelmingly released. The gas bubbles grow among the surfactant molecules, expanded by the generated CO₂ pressure. The SDS is an anionic surfactant consisting of a hydrophilic (water-loving) head and a hydrophobic (water-hating) tail. It was suggested that as the gas bubbles expand, the hydrophobic tail of each surfactant molecule is attracted to the CO₂ gas phases while their hydrophilic head is attracted to the water phase. Therefore, a bubble carrier system comprising a water film containing insulin and DTPA is self-assembled between double layers of surfactant molecules (SDS). The gas bubbles continue to expand until they come in contact with the mucosal layer covering the intestinal wall and eventually burst and release their loads of insulin (a model API in this experimental run; other APIs are also applicable), DTPA, and SDS. In the mucosal layer, DTPA serves as a paracellular enhancer for delivering insulin molecules through the epithelial cells, and the amphiphilic surfactant (SDS) enhances their paracellular and transcellular permeability. Both DTPA and SDS also protect insulin molecules by functioning as protease inhibitors.

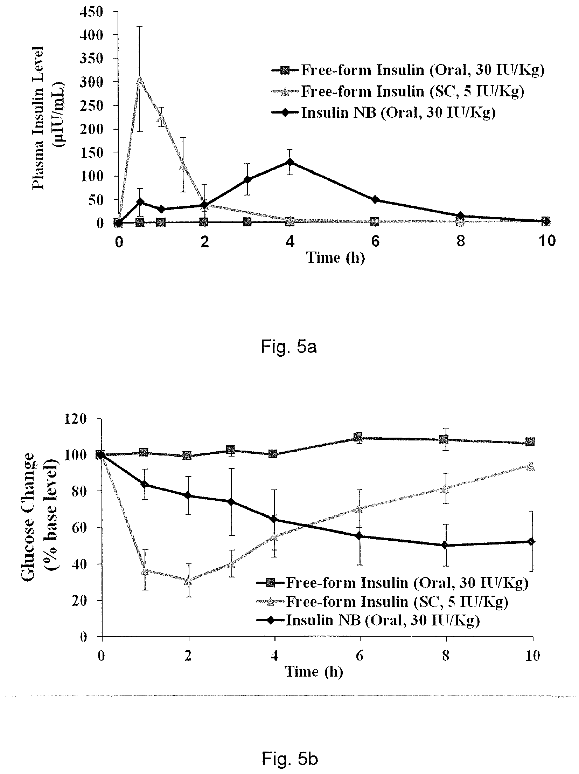

It is hypothesized that the bubble carrier system can mediate temporary alterations in the morphology of epithelial cell membranes and transient opening of their apical junctional complexes (AJCs), which facilitates the encapsulated insulin molecules in crossing the epithelial barrier and eventually being absorbed into systemic circulation, where they exert glucose-regulating effects as supported by Fig. 5b.

The DTPA dianhydride, SBC (a type of foaming agent), SDS, and insulin were thoroughly mixed and loaded in hard gelatin capsules. In one embodiment, the capsules were then coated with an enteric polymer (Eudragit® L100-55), which is dissoluble above pH 5.5 for protecting the loaded contents from acidic gastric fluid. The control was enteric-coated capsules without SBC.

***In vitro* dissolution of test capsules.** The dissolution of the enteric-coated capsules with or without SBC was evaluated *in vitro* in conditions simulating the pH environments in the gastrointestinal (GI) tract. In this experiment, Trypan blue was included in the test capsules as a dye indicator. The generation of CO₂ bubbles was investigated using an ultrasound machine. According to Fig. 8(a), both test capsules remained intact at pH 2.0 (mimicking the acidic milieu in the stomach), confirming the gastroresistant feature of Eudragit® L100-55. In the pH environments of the small intestine (pH 6.6 to pH 7.4), the load diffused through the pore that developed as the enteric polymer coating on the control capsule (containing no SBC) slowly dissolved. The absence of ultrasound signals in this control case suggests that no bubbles were generated.

Conversely, following partial dissolution of the enteric polymer and penetration of water into the gelatin capsule containing DTPA dianhydride and SBC, gas bubbles were immediately produced (Fig. 8(b)). When DTPA dianhydride is exposed to water, an acidic environment is produced, in which SBC decomposes to form CO₂ bubbles that are hyperechogenic and could act as a contrast enhancer for ultrasound imaging. The resulting gas pressure forced the capsule to rupture and quickly released its loaded contents. The invention preferably provides a capsule for oral delivery to an animal subject, the capsule comprising a gas generating agent or effervescent agent that causes the gas generating or effervescent agent to produce or generate a gas upon hydrolysis, wherein the gas generating agent is selected from the group consisting of ammonium bicarbonate, sodium bicarbonate, potassium bicarbonate, and calcium bicarbonate, wherein the gas is carbon dioxide.

**Formation of bubble carriers.** To identify their locations within the generated gas bubbles, insulin was labeled with a green fluorescence dye (fluorescein isothiocyanate, FITC) while DTPA was marked with a red fluorescence dye (cyanine 3, Cy3); the test samples were then imaged under a fluorescence microscope. Structural changes caused by expansion of the gas bubbles were observed by transmission electron microscopy (TEM) and by small angle X-ray scattering (SAXS).

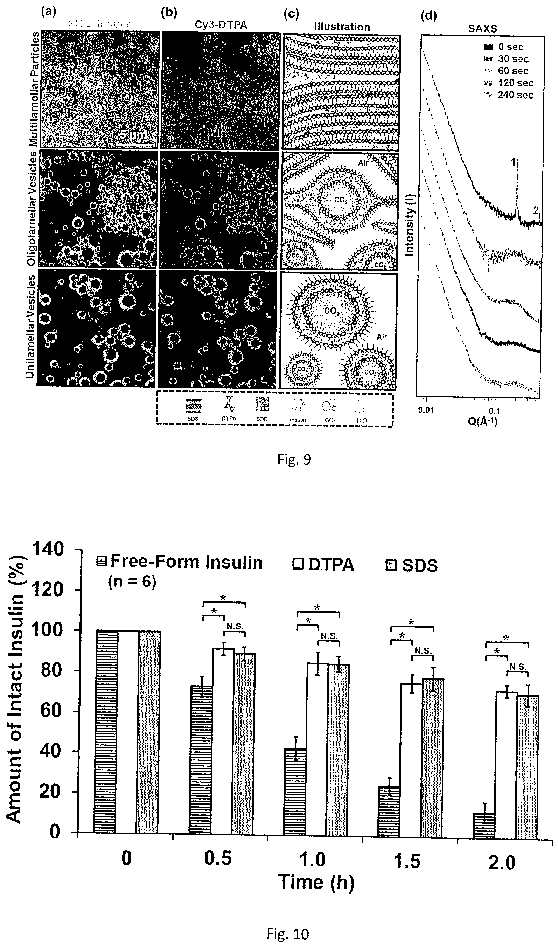

Conversely, as the contents of the capsule with the foaming agent were released and exposed to water, CO₂ gas bubbles in nanoscale were self-assembled (Fig. 2). The obtained TEM and fluorescence micrographs demonstrate that the gas bubbles grew from nanoscale to microscale (Fig. 9a and 9b) among the surfactant molecules (SDS) owing to the expansion of the generated CO₂. The gas bubbles were surrounded by a layer (thickness, approximately 150 nm) of FITC-insulin (Fig. 9a) and Cy3-DTPA (Fig. 9b), respectively. As the gas bubbles expanded, the hydrophilic heads of SDS remained solvated in the water phase, and the hydrophobic tails were extended into the CO₂ or air phases which are generally considered hydrophobic, as schematically illustrated in Fig. 9c. Thus, a self-assembled film of water sandwiched between the two layers of surfactant molecules stabilized the generated gas bubbles; the self-assembled water film could also serve as a colloidal carrier to carry the hydrophilic insulin and DTPA molecules. The locations of insulin and DTPA after bubble formation may be a factor for their subsequent delivery.

Fig. 9d shows the results of time-resolved SAXS performed to reveal the structural changes in gas bubbles during their formation. When the SDS in powder form came in contact with water (t = 0 sec, first row in Fig. 9a-9c), the SAXS profile showed two sharp peaks with the position ratio of 1:2, signifying that the surfactant molecules in the powder had self-assembled into a multilamellar structure with an interlamellar distance of ca. 4.0 nm. At 30 sec after reaction, the original primary peak transformed into a broad halo and the second-order peak vanished. This result indicates that the multilamellar structure was disrupted by the generated CO₂ bubbles to yield oligolamellar carriers composed of gas bubbles surrounded by water films (second row in Fig. 9a-9c). The progressive diminishment of the primary peak in the SAXS profiles suggested that the number of layers of surfactant molecules decreased as the reaction proceeded. After 240 sec, the SAXS peaks were no longer visible, which indicated the formation of unilamellar carriers (third row, Fig. 9a-9c).

**Enhanced epithelial permeability.** Drug permeation may occur across the intestinal epithelium and through either transcellular or paracellular routes. To elucidate the precise mechanisms of interactions between the bubble carrier system and epithelial cells and its subsequent transport of the drug, the effects of DTPA and SDS on epithelial permeability were individually studied using Caco-2 cell monolayers. Monolayers of Caco-2 cells derived from human colorectal adenocarcinoma are widely used in *in vitro* models for predicting intestinal drug permeability.

The AJC, which is comprised of adherens junctions (AJs) and tight junctions (TJs), is an important regulator of the intestinal cell structure and the epithelial barrier function. The AJs cause plasma membranes to combine, and they enhance TJ assembly; alteration of AJs therefore modulates the TJ structure and epithelial permeability. Claudin-4 (CLDN4) is a TJ protein whereas E-cadherin, a calcium (Ca²⁺)-dependent adhesion molecule, is the major transmembrane protein of AJs. Accordingly, extracellular Ca²⁺ is essential for formation of AJC and maintenance of cell-cell junctions. Both AJs and TJs can rapidly disassemble and reorganize in response to various extracellular stimuli.

**Inhibition of proteolytic degradation.** Inhibiting the proteolytic degradation of insulin in the intestinal fluid and the mucosal layer is critical for increasing its oral bioavailability. The inhibiting effects of DTPA and SDS on intestinal proteases were separately studied in *in vitro* studies of the proximal portion of the small intestine (*i.e.,* the duodenum) freshly isolated from rats. The various digestive enzymes secreted from the pancreas into the duodenum are well-recognized. The addition of DTPA or SDS in insulin produced a strong protective effect against the intestinal proteases. Insulin is highly sensitive to trypsin and chymotrypsin, which are Ca²⁺-dependent enzymes in the intestinal fluid and in the mucosal layer. One strategy for inhibiting intestinal protease activities is using complexing agents such as DTPA to remove essential metal ions (*e.g.,* Ca²⁺) from the enzyme structure at vicinity of the bioactive agent. A previous study indicated that SDS has a binding affinity for both free enzymes and the enzyme-substrate binary complex. Proposed molecular mechanisms of proteolytic inhibition by SDS include local conformational changes or altered charge distributions of the proteases induced by their specific binding of SDS.

***In vivo* dissolution of the test capsule and its subsequent release of insulin.** In the *in vivo* dissolution study, the capsule with SBC was orally administered to rats using a gavage needle; its counterpart containing no SBC served as a control. In this experiment, insulin was radiolabeled with Iodine-123 (¹²³I); additionally, a radiocontrast agent, barium sulfate, was incorporated in the test capsules. A single-photon emission computed tomography (SPECT)/computed tomography (CT) scanner was used for imaging. The dynamic plain X-ray and scintigraphy images of the rats were acquired sequentially at *2 min intervals* for 1 hour after oral administration of the test capsules. Fig. 11 shows that, once the capsule left the stomach, the CO₂ generated in the pH environment of the small intestine immediately disintegrated the SBC capsule; the ¹²³I-labeled insulin was then rapidly released (white arrow). During this time, the capsule without the foaming agent remained intact.

The bubble carriers formed with a water film containing insulin and DTPA sandwiched between two layers of SDS (Fig. 9). They then drifted in the intestinal lumen until they eventually bumped into the intestinal wall and burst, releasing their loaded insulin, DTPA, and SDS into the mucosal layer (Fig. 7). The released insulin, DTPA, and SDS then infiltrated the mucosal layer and accessed the epithelium surface. The presence of green fluorescence (FITC-insulin) on the lacteal side of the villi demonstrates that DTPA and SDS protect insulin molecules from proteolytic degradation and enhance their epithelial permeability and absorption into systemic circulation. Notably, the green fluorescence intensity was higher in the group treated with the SBC capsule compared to the group treated with the control capsule containing no SBC, indicating that the developed bubble carrier system is a promising vehicle for orally administered insulin.

***In vivo* pharmacokinetic (PK) and pharmacodynamic (PD) profiles.** The PK and PD profiles of the diabetic rats that received the capsules with or without SBC were analyzed. The control groups received free-form insulin solution *via* subcutaneous injection or the capsule containing the free-form insulin powder alone *via* oral ingestion. Fig. 5a and Fig. 5b show that subcutaneous injection of free-form insulin solution resulted in a maximum plasma concentration at 1 hour post administration, which caused a sharp decrease in the blood glucose level within 1-3 hours before returning to its basal level. As expected, oral administration of the capsule filled with the free-form insulin powder produced no hypoglycemic effects, indicating the poor oral absorption of insulin in the absence of a suitable delivery system.

Conversely, administration of the capsules containing protease inhibitors/absorption enhancers (DTPA and SDS) produced a slow but prolonged reduction in blood glucose levels, which indicated the continuing pharmacological activity of the absorbed insulin. Compared with the group given the capsule without SBC, the group given the capsule with SBC revealed a faster and enhanced absorption of insulin, resulting in a more sustained hypoglycemic effect. For the cases without and with SBC, Table 1 shows that the area-under-curve (AUC; 0-10 h) values were 123.8 ± 28.4 and 249.2 ± 34.1 µIU h/mL and the corresponding relative bioavailability (BA_{R}) values were 10.8 ± 3.1 and 21.7 ± 1.7% (*P* < 0.05), respectively.

**Gas generating agent in capsules:** The gas generating agent (may also be known as gas producing agent) includes ammonium bicarbonate, sodium bicarbonate, potassium bicarbonate or calcium bicarbonate. The invention relates to a capsule having a gas generating agent or effervescent agent, and optionally excipients. Upon hydrolysis or water penetrating into the capsule, the released proton from acid initiators reacts with the bicarbonate component to produce CO₂ gas inside the capsule and rupture the capsule or disturbing the contents (capsule payload) of the capsule. The chemical reaction for CO₂ gas production is illustrated below as reference.

NaHCO₃ + H⁺ → Na⁺ + H₂CO₃

H₂CO₃ → H₂O + CO₂

**A pharmaceutical composition comprising a nanobubble foaming system in capsules.** Fig. 7 shows the schematic representation of a bubble or nanobubble forming system. As illustrated with the nano-bubbling forming composition in a pharmaceutical composition of the present invention inside an enteric-coated capsule, the contact of intestinal fluid at a pH higher than the pH of the enteric-coating polymer enables the intestinal fluid into effervescent agent and leads to CO₂ generation, which subsequently makes the bioactive agent-loaded nanobubbles to puncture or rupture or push out the contents from the capsules instantly.

Fig. 12 shows chemical structures of a pharmaceutical composition comprising a gas generating agent (effervescent), an acid initiator (i.e., a compound causing the gas generating agent to produce gas) and a nanobubble forming agent (for example, sodium dodecyl sulfate SDS). It was suggested that the SDS or the like may serve to maintain the bubble structure, enhance transcellular permeation and/or enhance paracellular permeation. It was further suggested that an acid initiator, such as DTPA dianhydride, may serve to enhance paracellular permeation, and inhibit/reduce enzymatic reaction in GIT. The invention also relates to a pharmaceutical composition comprising a capsule that is loaded with a gas generating agent and a compound causing the gas generating agent to produce and release the gas upon hydrolysis. Preferably, the payload inside the capsule further comprises a nanobubble forming agent, a surfactant such as SDS, and a bioactive agent.

The nanobubble carrier system (to be loaded in a capsule for delivering to an animal subject) of the invention demonstrates a self-assembling characteristic with a bubble generation process. The first step of chemical reaction process is to produce CO₂ (gas).

Although the present invention has been described with reference to specific details of certain embodiments thereof, it is not intended that such details should be regarded as limitations upon the scope of the invention.

**Table 1. The PK parameters of insulin in diabetic rats given varying insulin formulations.**

| | Free-Form Insulin (SC) | Free-Form Insulin (Oral) | w/o Foaming Agent (Oral) | w/ Foaming Agent (Oral) |
|---|---|---|---|---|
| Dose (IU/kg) | 5.0 | 30.0 | 30.0 | 30.0 |
| Cₘₐₓ (µIU/mL) | 119.6 ± 3.8 | 1.8 ± 0.7 | 34.0 ± 6.4 | 68.4 ± 6.8 |
| Tₘₐₓ (h) | 1.0 | 5.0 | 5.0 | 4.0 |
| AUC_{(0-10 h)} (µIU h/mL) | 191.3 ± 30.7 | 6.5 ± 2.7 | 123.8 ± 28.4 | 249.2 ± 34.1 |
| BA_{R} (%) | 100 | 0.5 ± 0.3 | 10.8 ± 3.1 | 21.7 ± 1.7 |

| | | | | |
|---|---|---|---|---|
| Oral: oral administration of test capsules; SC: subcutaneous injection; Cₘₐₓ: maximum plasma concentration; Tₘₐₓ: time at which Cₘₐₓ is attained; AUC_{(0-10 h)}: area under the plasma concentration-time curve; BA_{R}: relative bioavailability of insulin (n = 6). | | | | |

## Claims

1. A pharmaceutical composition suitable for oral administration to an animal subject, comprising
a gas generating ingredient, a surfactant, an acid initiator and at least one bioactive agent, wherein
said pharmaceutical composition is loaded within an enteric-coated capsule,
said gas generating ingredient is selected from the group consisting of ammonium bicarbonate, sodium bicarbonate, potassium bicarbonate, and calcium bicarbonate, and
said acid initiator reacts with said gas generating ingredient in an intestinal tract of the animal subject to produce a gas which is carbon dioxide upon contacting water, and
said acid initiator is an anhydride of complexone, said complexone being selected from the group consisting of DTPA (diethylene triamine pentaacetic acid), EDTA (ethylene diamine tetra acetate), IDA (iminodiacetic acid), NTA (nitrilotriacetic acid), EGTA (ethylene glycol tetraacetic acid), BAPTA (1,2-bis(o-aminophenoxy)ethane-N,N,N',N'-tetraacetic acid), DOTA (1,4,7,10-tetraazacyclododecane-N,N',N,N'-tetraacetic acid), and NOTA (2,2',2"-(1,4,7-triazonane-1,4,7-triyl)triacetic acid).

2. The pharmaceutical composition according to claim 1, wherein said capsule is made of gelatin or methyl cellulose selected from the group consisting of hydroxypropyl methyl cellulose (HPMC), carboxymethyl cellulose, hydroxyethyl methyl cellulose (HEMC), and methyl cellulose derivatives.

3. The pharmaceutical composition according to any preceding claim, further comprising at least one excipient.

4. The pharmaceutical composition according to claim 3, wherein said at least one excipient is selected from the group consisting of a pharmaceutically acceptable carrier, diluent, desiccant, solubilizer, absorption enhancer, and emulsifier.

5. The pharmaceutical composition according to claim 4, wherein said absorption enhancer is selected from the group consisting of bile salts, medium-chain fatty acids, phosphate esters, chitosan, and chitosan derivatives.

6. The pharmaceutical composition according to claim 1, wherein said surfactant is sodium dodecyl sulfate, polyoxyethylene sorbitan monostearate, or sodium dodecyl benzene sulfonate, or wherein said surfactant is an anionic surfactant, cationic surfactant, amphoteric or non-ionic surfactant.

7. The pharmaceutical composition according to any preceding claim, wherein said at least one bioactive agent is an anti-diabetic compound, wherein said anti-diabetic compound is selected from the group consisting of insulin, GLP-1, an insulin sensitizer, an insulin secretagogue, an inhibitor of dipeptidyl peptidase 4, metformin, alpha-glucosidase inhibitors, sodium-glucose co-transporter type 2 (SGLT2) inhibitors, benfluorex, tolrestat, mitofusin 1, mitofusin 2, and combinations thereof.

8. The pharmaceutical composition according to any one of claims 1 to 6, wherein said at least one bioactive agent is selected from the group consisting of proteins, peptides, nucleosides, nucleotides, antiviral agents, antineoplastic agents, antibiotics, oxygen-enriching agent, oxygen-containing agent, growth hormone, human growth hormone, heparin, low molecular weight heparin, anti-coagulating agents, anti-hemophilic factor, anti-epileptic drug, Alzheimer's antagonist, anti-inflammatory drugs, calcitonin, cyclosporin, erythropoietin, erythropoiesis-stimulating agent, oxytocin, tyrosine, enkephalin, tyrotropin releasing hormone, follicle stimulating hormone, luteinizing hormone, vasopressin, catalase, superoxide dismutase, interleukin-11, interferon, colony stimulating factor, granulocyte colony-stimulating factor, tumor necrosis factor, tumor necrosis factor inhibitor, melanocyte-stimulating hormone, antibodies, and monoclonal antibodies.

9. The pharmaceutical composition according to any preceding claim, wherein the complexone is DTPA and the surfactant is sodium dodecyl sulfate.

10. A pharmaceutical composition of any preceding claim for use in medicine.

## Patentansprüche

1. Pharmazeutische Zusammensetzung, geeignet zur oralen Verabreichung an ein Tiersubjekt, umfassend
einen gaserzeugenden Inhaltsstoff, ein grenzflächenaktives Mittel, einen Säureinitiator und wenigstens ein bioaktives Mittel, wobei die pharmazeutische Zusammensetzung in eine magensaftresistent beschichtete Kapsel geladen ist,
wobei der gaserzeugende Inhaltsstoff ausgewählt ist aus der Gruppe bestehend aus Ammoniumbicarbonat, Natriumbicarbonat, Kaliumbicarbonat und Calciumbicarbonat und
der Säureinitiator mit dem gaserzeugenden Inhaltsstoff in dem Darmtrakt des Tiersubjekts bei Inkontaktkommen mit Wasser reagiert, um ein Gas zu erzeugen, das Kohlendioxid ist, und
der Säureinitiator ein Anhydrid eines Komplexons ist, wobei das Komplexon ausgewählt ist aus der Gruppe bestehend aus DTPA (Diethylentriaminpentaessigsäure), EDTA (Ethylendiamintetraacetat), IDA (Iminodiessigsäure), NTA (Nitrilotriessigsäure), EGTA (Ethylenglycoltetraessigsäure), BAPTA (1,2-Bis(o-aminophenoxy)ethan-N,N,N',N'-tetraessigsäure, DOTA (1,4,7,10-Tetraazacyclododecan-N,N,N',N'-tetraessigsäure) und NOTA (2,2',2"-(1,4,7-Triazonan-1,4,7-triyl)triessigsäure).

2. Pharmazeutische Zusammensetzung gemäß Anspruch 1, wobei die Kapsel aus Gelatine oder Methylcellulose ausgewählt aus der Gruppe bestehend aus Hydroxypropylmethylcellulose (HPMC), Carboxymethylcellulose, Hydroxyethylmethylcellulose (HEMC) und Methylcellulosederivaten besteht.

3. Pharmazeutische Zusammensetzung gemäß einem der vorstehenden Ansprüche, ferner umfassend wenigstens einen Hilfsstoff.

4. Pharmazeutische Zusammensetzung gemäß Anspruch 3, wobei der wenigstens eine Hilfsstoff ausgewählt ist aus der Gruppe bestehend aus einem pharmazeutisch verträglichen Träger, Verdünnungsmittel, Trockenmittel, Solubilisierungsmittel, Absorptionsverstärker und Emulgator.

5. Pharmazeutische Zusammensetzung gemäß Anspruch 4, wobei der Absorptionsverstärker ausgewählt ist aus der Gruppe bestehend aus Gallensalzen, Fettsäuren mit mittlerer Kettenlänge, Phosphatestern, Chitosan und Chitosanderivaten.

6. Pharmazeutische Zusammensetzung gemäß Anspruch 1, wobei das grenzflächenaktive Mittel Natriumdodecylsulfat, Polyoxyethylensorbitanmonostearat oder Natriumdodecylbenzolsulfonat ist,
oder wobei das grenzflächenaktive Mittel ein anionisches grenzflächenaktives Mittel, kationisches grenzflächenaktives Mittel, amphoteres oder nichtionisches grenzflächenaktives Mittel ist.

7. Pharmazeutische Zusammensetzung gemäß einem der vorstehenden Ansprüche, wobei das wenigstens eine bioaktive Mittel eine antidiabetische Verbindung ist, wobei die antidiabetische Verbindung ausgewählt ist aus der Gruppe bestehend aus Insulin, GLP-1, einem Insulinsensibilisator, einem Insulinsekretanalogon, einem Hemmer der Dipeptidylpeptidase 4, Metformin, alpha-Glucosidase-Hemmern, Hemmern des Natriumglucose-cotransporters Typ 2 (SGLT2), Benfluorex, Tolrestat, Mitofusin 1, Mitofusin 2 und Kombinationen davon.

8. Pharmazeutische Zusammensetzung gemäß einem der Ansprüche 1 bis 6, wobei das wenigstens eine bioaktive Mittel ausgewählt ist aus der Gruppe bestehend aus Proteinen, Peptiden, Nukleosiden, Nulkeotiden, antiviralen Mitteln, antineoplastischen Mitteln, Antibiotika, sauerstoffanreichernden Mitteln, sauerstoffhaltigen Mitteln, Wachstumshormon, menschlichem Wachstumshormon, Heparin, Heparin mit niedrigem Molekulargewicht, Antikoagulationsmitteln, antihämophilem Faktor, antiepileptischen Arzneimitteln, Alzheimer-Antagonisten, entzündungshemmenden Arzneimitteln, Calcitonin, Cyclosporin, Erythropoetin, Erythropoesestimulierenden Mitteln, Oxytocin, Tyrosin, Enkephalin, Tyrotropin-freisetzendem Hormon, follikelstimulierendem Hormon, Luteinisierungshormon, Vasopressin, Katalase, Superoxiddismutase, Interleukin-11, Interferon, koloniestimulierendem Faktor, Granulozytenkoloniestimulierendem Faktor, Tumornekrosefaktor, Tumornekrosefaktor-Hemmer, melanozytenstimulierendem Hormon, Antikörpern und monoklonalen Antikörpern.

9. Pharmazeutische Zusammensetzung gemäß einem der vorstehenden Ansprüche, wobei das Komplexon DTPA ist und das grenzflächenaktive Mittel Natriumdodecylsulfat ist.

10. Pharmazeutische Zusammensetzung gemäß einem der vorstehenden Ansprüche zur Verwendung in der Medizin.

## Revendications

1. Composition pharmaceutique appropriée pour une administration orale à un sujet animal, comprenant un ingrédient de génération de gaz, un tensioactif, un initiateur acide et au moins un agent bioactif, dans laquelle ladite composition pharmaceutique est chargée au sein d'une gélule à enrobage entérique, ledit infrédient de génération de gaz est choisi dans le groupe constitué par du bicarbonate d'ammonium, du bicarbonate de sodium, du bicarbonate de potassium et du bicarbonate de calcium, et ledit initiateur acide réagit avec ledit ingrédient de génération de gaz dans un tractus intestinal du sujet animal pour produire un gaz qui est du dioxyde de carbone lors de la mise en contact avec de l'eau, et
ledit initiateur acide est un anhydride de complexone, ladite complexone étant choisie dans le groupe constitué par le DTPA (acide pentaacétique de diéthylène triamine) l'EDTA (tétraacétate d'éthylène diamine), l'IDA (acide iminodiacétiqueà, le NTA (acide nitriloacétique), l'EGTA (acide tétraacétique d'éthylène glycol), le BAPTA (acide 1,2-bis(o-aminophénnoxy)éthane-N,N-N',N'-tétraacétique), le DOTA (acide 1,4,7,10-tétraazacyclododécane-N,N',N,N'-tétraacétique) et le NOTA (acide 2,2',2"-(1,4,7-triazonane-1,4,7-triyl)triacétique).

2. Composition pharmaceutique selon la revendication 1, dans laquelle ladite gélule est en gélatine ou en méthylcellulose choisie dans le groupe constitué par l'hydroxypropylméthylcellulose (HPMC), la carboxyméthylcellule, l'hydroxyéthylméthylcellulose (HEMC) et des dérivés de la méthylcellulose.

3. Composition pharmaceutique selon l'une quelconque des revendications précédentes, comprenant en outre au moins un excipient.

4. Composition pharmaceutique selon la revendication 3, dans laquelle ladite au moins un excipient est choisi dans le groupe constitué par un véhicule, un diluant, un dessiccant, un agent de solubilisation, un améliorateur d'absorption et un agent émulsifiant.

5. Composition pharmaceutique selon la revendication 4, dans laquelle ledit améliorateur d'absorption est choisi dans le groupe constitué par les sels biliaires, les acides gras à chaîne moyenne, les esters de phosphate, le chitosane et les dérivés de chitosane.

6. Composition pharmaceutique selon la revendication 1, dans laquelle ledit tensioactif est le dodécylsulfate de sodium, le monostéarate de polyoxyéthylène sorbitane ou le dodécylbenzènesulfonate de sodium,
Ou dans laquelle ledit tensioactif est un tnesioactif anionique, un tensioactif cationique, un tensioactif amphotère ou non ionique.

7. Composition pharmaceutique selon l'une quelconque des revendications précédentes, dans laquelle ledit au moins un agent bioactif est un composé anti-diabète, dans laquelle ledit composé anti-diabétique est choisi dans le groupe constitué par l'insuline, le GPL-1, un sensibilisateur à l'insuline, un sécrétagogue de l'insuline, un inhibiteur de la dipeptidyl peptidase 4, de la metformine, des inhibiteurs de l'alpha-glucosidase, un analogue de l'amyline, des inhibiteurs du co-transporteur sodium-glucose de type 2 (SGLT2), du benfluorex, du tolrestat, de la mitofusine 1, de la mitofusine 2, et des combinaisons de ceux-ci.

8. Composition pharmaceutique selon l'une quelconque des revendications 1 à 6, dans laquelle ledit au moins un agent bioactif est choisi dans le groupe constitué par les protéines, les peptides, les nucléosides, les nucléotides, les agents antiviraux, les agents antinéoplasiques, les antibiotiques, un agent enrichissant en oxygène, un agent contenant de l'oxygène, l'hormone de croissance, l'hormone de croissance humaine, l'héparine, une héparine de bas poids moléculaire, les agents anticoagulants, le facteur antihémophilique, un médicament anti-épileptique, un antagoniste d'Alzheimer, les médicaments anti-inflammatoires, la calcitonine, la cyclosporine, l'érythropoïétine, un agent stimulant l'érythropoïèse, l'oxytocine, la tyrosine, l'enképhaline, l'hormone libérant la tyrotropine, l'hormone folliculo-stimulante, l'hormone lutéinisante, la vasopressine, la catalase, la superoxyde dismutase, l'interleukine-11, l'interféron, le facteur stimulant les colonies, facteur stimulant les colonies de granulocytes, le facteur de nécrose tumorale, un inhibiteur de la nécrose tumorale, le facteur de nécrose tumorale , l'hormone stimulant les mélanocytes, les anticorps et les anticorps monoclonaux.

9. Composition pharmaceutique selon l'une quelconque des revendications précédentes, dans laquelle la complexone est le DTPA et le tensioactif est le dodécylsulfate de sodium.

10. Composition pharmaceutique selon l'une quelconque des revendications précédentes pour utilisation en médecine.
